# EUROPEAN PATENT APPLICATION

(11) **EP 2 420 237 A1**
(43) Date of publication of application: **22.02.2012**
(21) Application number: 10397511.6
(22) Date of filing: 11.08.2010
(51) Int. Cl.: A61K 31/47, A61P 25/16

(54) **Fluorinated derivatives of endogenous isoquinolines for use in the treatment of diseases mediated through endogenous isoquinoline pathways**

(71) Applicant: Takio Ville, 03400 Vihti (FI); Ekholm, Matti, 00410 Helsinki (FI)
(72) Inventor: Takio Ville, 03400 Vihti (FI); Ekholm, Matti, 00410 Helsinki (FI)
(74) Representative: Syvänen, Ralf Ossian

(57) **Abstract**

This invention relates to a series of new fluorinated endogenous isoquinoline-mimicking compounds intended for use in the process of preparing valuable dietary supplements and pharmaceuticals as well as research tools for a wide range of medical, psychiatric and/or neurological conditions. The new compounds can be synthesized using well documented reactions and commercially available starting materials.

## Description

### Summary of the invention

This invention relates to a series of new fluorinated derivatives of endogenous isoquinolines, some of which are useful as general neuroprotectants, antiparkinsonians, antiaddictives, analgesics, anticonvulsants, antidepressants and mood stabilizers as well as tools in biochemistry research. These derivatives work by mimicking and modulating endogenous isoquinolines and their pharmacological responses.

### Description of the drawings

FIG. 1 Some of the known metabolic pathways of TIQ derivatives
FIG. 2 The main differences in metabolism between TIQ and 1MeTIQ
FIG. 3 Well documented pathways for the synthesis of TIQ derivatives

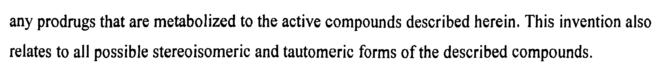

FIG. 4 Illustration of the backbone structures of the compounds and locations of possible substituents

### Background of the invention

Endogenous isoquinolines are formed by condensation of biogenic amines - such as phenethylamine - and simple aldehydes such as formaldehyde or acetaldehyde. They are known to modulate neurotransmission, central metabolism and motor activity. An endogenous TIQ derivative salsolinol (1-methyl-1,2,3,4-tetrahydroisoquinoline-6,7-diol) is considered to be a causative factor of Parkinson's disease (PD), while (R)-1-MeTIQ (1-methyl-1,2,3,4-tetrahydroisoquinoline) was shown to posses an antiparkinsonian activity. Until recently 1MeTIQ was the only known neuroprotective / PD preventing TIQ derivative. In 2006 Katsuhiro OKUDA et al. discovered that 5-/6-/7-monohydroxylated 1MeTIQ derivatives are neuroprotective and PD preventing indeed, even more so than the parent compound.

It has been demonstrated that concentrations of many endogenous TIQ derivatives are significantly elevated in the urine and cerebrospinal fluid of PD / ADHD patients compared to controls, the content of 1MeTIQ however is significantly decreased in PD patients cerebrospinal fluid and brain.

Salsolinol is formed enzymatically as well as non-enzymatically as a condensation product of acetaldehyde - the primary metabolite of ethanol - with dopamine in the brain of mammals. Salsolinol affects the uptake of catecholamines into nerve terminals, release of stored catecholamines, activity of monoamine oxidase (MAO) and catechol-O-methyl transferase (COMT) and activaty of tyrosine hydroxylase. Ethanol induced elevation of salsolinol levels is known to participate in the development of ethanol addiction / alcoholism.

Most tetrahydroisoquinolines penetrate to the brain in pharmacologically relevant amounts and induce a variety of effects. Most of the TIQ and 1MeTIQ exit the brain (90.4% and 95.3%) and is excreted in urine (76 % and 72 %) unchanged. The hydroxylated (C4 at isoquinoline backbone = R₅) derivatives of TIQ and 1MeTIQ were the most abundant metabolites in the urine (2.7% 8.7%).

### Detailed description of the invention

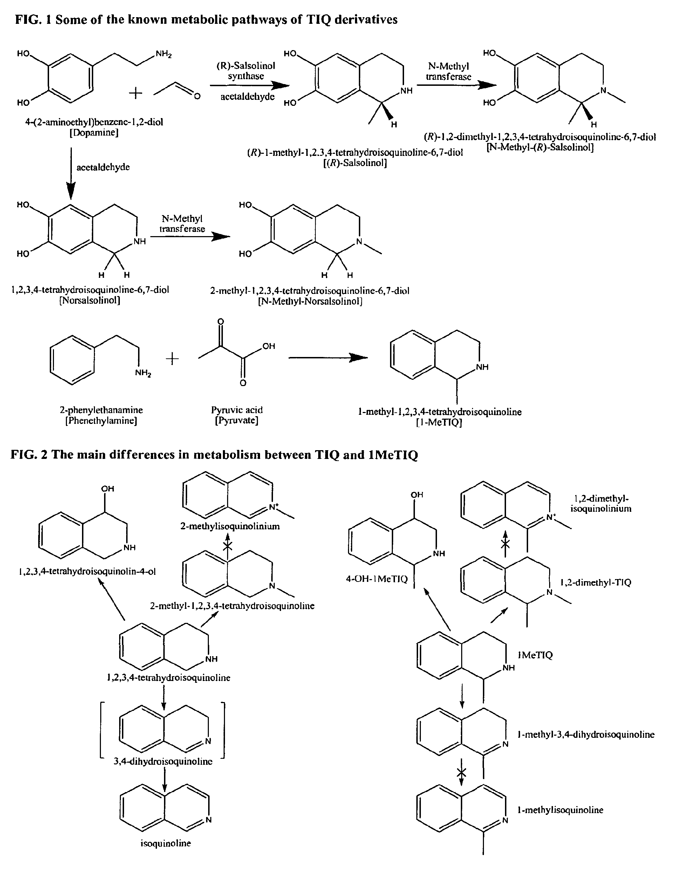

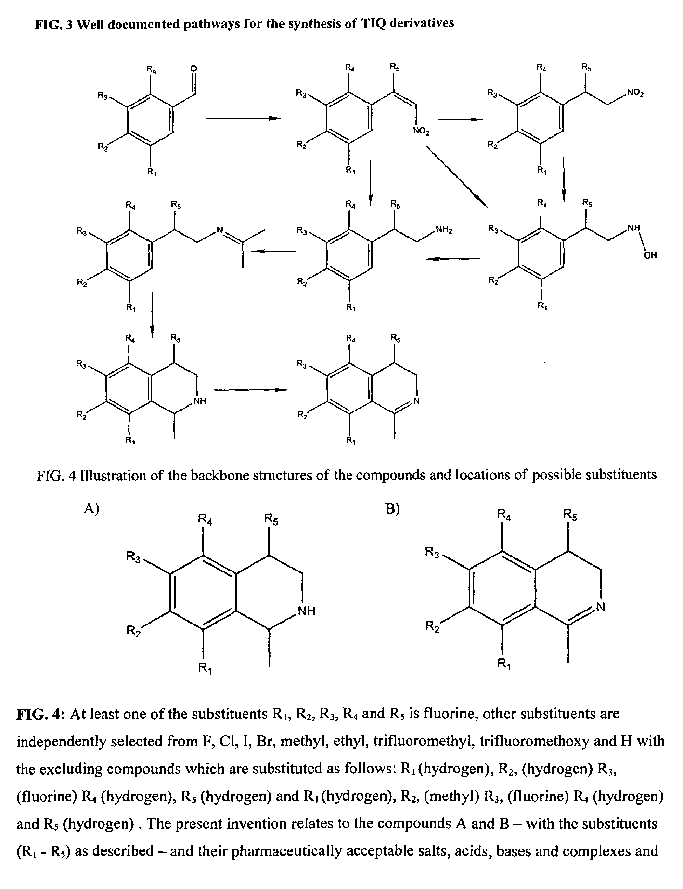

Endogenous isoquinolines are compounds which are formed by condensation of biogenic amines (FIG. 1 and FIG. 2) and simple aldehydes (such as formaldehyde or acetaldehyde). The compounds of the invention can be synthesized using well documented reactions (FIG. 3) and commercially available starting materials. We have explored a series of new TIQ derivatives primarily targeted to mimick the action of 1MeTIQ. These novel 1MeTIQ compounds can be used to achieve many desirable pharmacoligical responses. Fluorination can alter the bond strength, lipophilicity, conformation, electrostatic potential, dipoles and pKa.

Fluorination at the position of metabolic attack - mainly at R₅ to be exact - is used to alter the route and rate of metabolic degradation. Fluorination may also alter the tissue distribution, pharmacodynamics and toxicology of the compound. It can be generalized that substituting hydrogen with fluorine causes minimal steric effects at receptor.

These new compounds are closely related to 1MeTIQ and salsolinol. Some of these can be used to treat addictions in general - from alcohol to cocaine and heroin. Number of positive pharmacological responses can be achieved simultaneously. While decreasing the tendency to relapse and likelihood of developing an addiction, these compunds can act as general mood stabilizers and general neuroprotectants possessing remarkable antiparkinsonian and antiepileptic character.

The compounds exhibit many pharmacological responses, such as
- prolonging the duration of morphine without enhancing the peak action
- antagonizing the development of morphine tolerance
- reducing the naloxone-precipitated withdrawal symptoms
- inhibiting the reinstatement of cocaine self-administration
- attenuating cravings
- inhibiting the activity of monoamine-oxidases (MAOs)
- inhibiting the activity of acetylcholinesterase (ACE)
- neuroprotection
- shifting the catabolism of catecholamine neurotransmitters towards catechol-O-methyl transferase (COMT) -dependent methylation
- inhibition or enhancement of the release of prolactin
- releasing norepinephrine
- inducing or inhibiting neuron related apoptosis and/or necrosis
- abolishing cocaine induced inhibition of noradrenalin metabolism

These new fluorinated derivatives of endogenous isoquinolines can be coupled with any amino acid or amino acid derivative to form a mixtures, complexes or prodrugs. For this purpose carnosine or N-acetylcarnosine are prerable because of their protective effects on salsolinol-mediated Cu,Zn-superoxide dismutase inactivation.

### Example of syntheses

Step 1. Knoevenagel Condensation - Tet. Lett. 39, 8013-8016 (1998):
   1.0 mol eq. subst. benzaldehyde, 1.2 mol eq. nitromethane, 0.47 mol eq. ammonium acetate and 0.35 mol eq. GAA was sonicated (40kHz) at RT for 3 h. After removal of nitromethane, partition between dichloromethane and water then brine gave crude product which was recrystallized from aq, (m)ethanol or AcOH.
   Or:
   1.0 mol eq. subst. benzaldehyde, 1.2 mol eq. nitromethane and 0.1 mol eq. cyclohexylamine was mixed and kept in dark for 4 weeks, or until H2O formation ceased. The crude product was ground, washed with brine and recrystallized from aq. (m)ethanol or AcOH.
   Or in the case offs halogen substitution (preferably fluorine), the synthesis proceeds via nitroalcohol intermediate, otherwise skip to step 4:
   1.0 mol eq. subst. benzaldehyde, with 1.0 mol eq. of triethylamine and 1.2 mol eq. nitromethane was stirred in methanol at -12 °C for 2½ h, and the amine quenched with 1.0 mol eq. of GAA while still freezing cold. Most of the solvent was stripped under vacuum, and the remains were dissolved in DCM and washed two times with water and once with brine. The DCM was stripped, leaving behind the crude nitroalcohol.
Step 2. (in the case of R₅ halogen substitution) Sulfonating the aliphatic OH-group to appropriate sulfonyl ester by stirring 1.0 mol eq. of subst. phenyl-2-amine-1-ol in DCM with 1.2 mol eq. of triethylamine (or using pyridine for the solvent) with and adding slowly 1.1 mol eq. of methylsulfonyl chloride maintaining the temperature at -5 °C until conversion was complete. The product was washed several times with brine, dried over anhydrous MgSO₄ and concentrated in vacuo.
Step 3. (in the case of R₅ halogen substitution) Modified Finkelstein reaction to the sulfonate ester with potassium halide (in this case KF) proceeded by dissolving 1 mol eq. of sulfonyl-intermediate from the step 2 with 6 mL of acetonitrile per gram of substrate, 0.5 mol eq. of 1-butyl-3-methylimidazolium tetrafluoroborate (bmim[bf4]) and 5 mol eq. of H2O which after 1.05 mol eq. amount of KF was added and the solution was mixed and sonicated at RT for 180 min or until TLC showed completion. The R₅-halogen substituted compounds were then extracted with DCM and washed several times with brine, dried over anhydrous MgSO₄ and concentrated in vacuo prior proceeding.
Step 4. Reduction of the possible C=C bond and nitro-group to the amino-group (well-known / obvious, hence the process and the workup are intentionally left out from this example section).
Step 5. Pictet-Spengler reaction - industrial scale (EP19970936381)
   1.0 mol eq. subst. N-tosyl-phenethylamine and 3.0 mol eq. boron trifluoroetherate was refluxed with 21.0 mol eq. of diethoxymethane for 12 h in N2 atmosphere or until TLC showed completion. The nitrogen is afterwards de-protected.
   Or:
   1.0 mol eq. subst. phenethylamine was refluxed with 3.0 mol eq. acetaldehyde and 1.2 mol eq. hydrochloric acid 37% until TLC showed completion.
Step 6. Workup
   The reaction mixture was partitioned between ethyl acetate (10 mL / 1 g substrate) and water (10 mL / 1 g substrate), separated, and the organic layer is washed twice with saturated sodium bicarbonate and dried over sodium sulfate (Na2S04). The drying agent was filtered off and the filtrate was distilled under reduced pressure to yield the desired compound.

The method of preparing 3,4-dihydroisoquinolines from 1,2,3,4-tetrahydroisoquinolines is well known and also described in US Patent 6034094.

The methods of preparation of pharmaceutically accepable salts and complexes or coupled compounds are also well known, hence the process is intentionally left out from this example section.

### References

1. Isolation of 1-methyl-1,2,3,4-Tetrahydroisoquinoline-Synthesizing Enzyme from Rat Brain: A Possible Parkinson's Disease-Preventing Enzyme; BIOCHEMICAL AND BIOPHYSICAL RESEARCH COMMUNICATIONS 236, 676-681 (1997)
2. 4-Hydroxylation of Debrisoquine by Human CYP1A1 and Its Inhibition by Quinidine and Quinine; THE JOURNAL OF PHARMACOLOGY AND EXPERIMENTAL THERAPEUTICS 301, 1025-1032 (2002)
3. Metabolism and penetration through blood-brain barrier of Parkinsonism-related compounds; DRUG METABOLISM AND DISPOSITION 19, 257-262 (1990)
4. Disposition of 1,2,3,4,-tetrahydroisoquinoline in the brain of male Wistar and Dark Agouti rats; BRAIN RESEARCH 996,168-179 (2004)
5. The interaction of tetrahydroisoquinoline derivatives with antinociceptive action of morphine and oxotremorine in mice; JOURNAL OF NEURAL TRANSMISSION 110, 1205-1213 (2003)
6. An endogenous neuroprotectant substance, 1-methyl-1,2,3,4-tetrahydroisoquinoline (IMeTIQ), prevents the behavioral and neurochemical effects of cocaine reinstatement in drug-dependent rats; JOURNAL OF NEURAL TRANSMISSION 114, 307-317 (2007)
7. In vivo formation of salsolinol induced by high acetaldehyde concentration in rat striatum employing microdialysis; ALCOHOL & ALCOHOLISM 38,197-201 (2003)
8. Protective Effects of Carnosine and N-Acetylcarnosine on Salsolinol-mediated Cu,Zn-superoxide Dismutase Inactivation; BULLETIN OF THE KOREAN CHEMICAL SOCIETY 28, 1881-1884 (2007)
9. Differential Cell Death Induced by Salsolinol with and without Copper: Possible Role of Reactive Oxygen Species; MOLECULAR PHARMACOLOGY 60, 440-49 (2001)
10. 3,4-Dihydroisoquinoline compounds as muscarinic receptor antagonists for the treatment of respiratory, urinary and gastrointestinal diseases; PCT WO 2006/035280 A1
11. Identification of salsolinol in the mediobasal hypothalamus of lactating ewes and its relation to suckling-induced prolactin and growth hormone release; JOURNAL OF ENDOCRINOLOGY, Epub ahead of print Apr 23. (2008)
12. Quantitative chiral analysis of salsolinol in different brain regions of rats genetically predisposed to alcoholism; JOURNAL OF CHROMATOGRAPHY 863, 206-214 (2008)
13. Effect of (R)-Salsolinol and N-Methyl-(R)-Salsolinol on the Balance Impairment Between Dopamine and Acetylcholine in Rat Brain: Involvement in Pathogenesis of Parkinson Disease; CLINICAL CHEMISTRY 54:4, 705-712 (2008)
14. Effects of single and repeated administration of 1,2,3,4-tetrahydroisoquinoline analogs on the binding of [11C]raclopride to dopamine D2 receptors in the mouse brain; JOURNAL OF NEURAL TRANSMISSION 108, 1111-1125 (2001)
15. The role of catecholamines in the prolactin release induced by salsolinol; NEUROCHEMISTRY INTERNATIONAL 51, 319-322 (2007)
16. Parkinsonism-Preventing Activity of 1-Methyl-1,2,3,4-tetrahydroisoquinoline Derivatives in C57BL Mouse in Vivo; BIOLOGICAL AND PHARMACEUTICAL BULLETIN 29,1401-1403 (2006)
17. Inhibition of rodent brain monoamine oxidase and tyrosine hydroxylase by endogenous compounds - 1,2,3,4-tetrahydroisoquinoline alkaloids; POLISH JOURNAL OF PHARMACOLOGY 56, 727-734 (2004)
18. Biphasic effects of acetaldehyde-biogenic amine condensation products on membrane fluidity; JOURNAL OF PHARMACY AND PHARMACOLOGY 53, 121-127 (2000)
19. Isoquinoline and dihydroisoquinoline 5-HT₃ receptor antagonists; US Patent 5491148

## Claims

1. The use of the compounds - in which the backbone structure is A or B (as shown below) 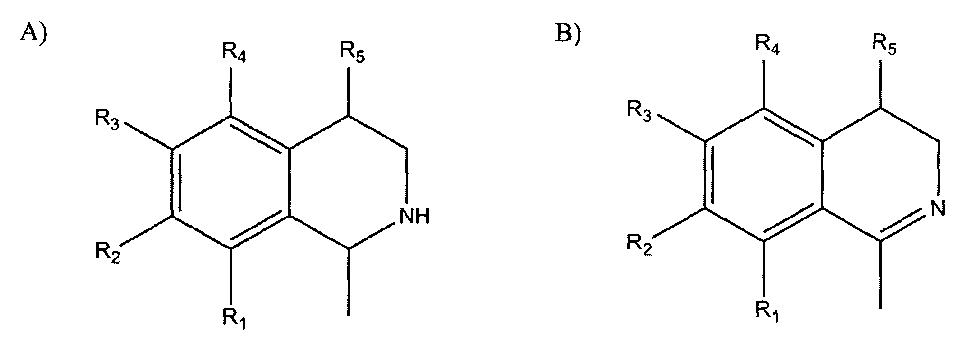 and at least one of the substituents R₁, R₂, R₃, R₄ and R₅ is fluorine, while other substituents are independently selected from fluorine, chloride, iodine, bromine, methyl, ethyl, trifluoromethyl, trifluoromethoxy, hydrogen, or deuteroatoms but excluding compounds which are substituted as follows: R₁ (hydrogen), R₂, (hydrogen) R₃, (fluorine) R₄ (hydrogen), R₅ (hydrogen) and R₁ (hydrogen), R₂, (methyl) R₃, (fluorine) R₄ (hydrogen) and R₅ (hydrogen) - in the process of preparing in vivo dietary supplements, treatments, medications, pharmaceuticals, formulations, carriers, diluents, vehicles, complexes, mixtures and/or coupled compounds (coupled compound = a prodrug which is metabolized in to an active compound described herein) - for the treatment of medical, psychiatric and/or neurological conditions and disorders such as, but not limited to, Alzheimer's disease, Parkinson's disease, major depression, minor depression, atypical depression, dysthymia, attention deficit disorder, attention-deficit hyperactivity disorder, hyperactivity, conduct disorder, narcolepsy, social phobia, obsessive-compulsive disorder, atypical facial pain, eating disorders, all conceivable types of cancer and/or tumors, drug withdrawal syndromes and drug dependence disorders - including but not limited to dependence and/or withdrawal from analgesics, anaesthetics, antidepressants, antipsychotics, anxiolytics, deliriants, dissociatives, empathogens, entactogens, entheogens, euphoricants, hypnotics, intoxicants, psychedelics and sedatives such as alcohol, opioids, amphetamines, cocaine, tobacco and cannabis, - melancholia, panic disorder, bulimia, anergic depression, treatment-resistant depression, headache, chronic pain syndrome, generalized anxiety disorder and other conditions in which these compounds could be used as therapeutics and/or research tools.
